# EUROPEAN PATENT SPECIFICATION

(11) **EP 4 203 950 B1**
(45) Date of publication and mention of the grant of the patent: **18.02.2026**
(21) Application number: 21783084.3
(22) Date of filing: 26.08.2021
(51) Int. Cl.: A61K 31/4192, A61K 39/00, A61K 31/675, A61K 31/704, A61K 31/475, A61K 31/573, A61K 9/00, A61P 35/00

(54) **COMBINATIONS OF A DHODH INHIBITOR AND R-CHOP THERAPY FOR THE TREATMENT OF DIFFUSE LARGE B-CELL LYMPHOMA, MANTLE CELL LYMPHOMA AND BURKITT LYMPHOMA**
KOMBINATIONEN EINES DHODH-INHIBITORS UND EINER R-CHOP-THERAPIE ZUR BEHANDLUNG VOM DIFFUSEN GROSSZELLIGEN B-ZELL-LYMPHOM, MANTELZELLLYMPHOM UND BURKITT-LYMPHOM
COMBINAISONS D'UN INHIBITEUR DE DHODH ET D'UNE THÉRAPIE R-CHOP POUR LE TRAITEMENT DES LYMPHOMES DIFFUS À GRANDES CELLULES B, À CELLULES DU MANTEAU, ET DE BURKITT

(30) Priority: 27.08.2020 US 202063070984 P
(43) Date of publication of application: 05.07.2023
(73) Proprietor: Les Laboratoires Servier, 92284 Suresnes Cedex (FR)
(72) Inventor: COCO, John, North Andover, MA 01845 (US); NICOLAY, Brandon, Manchester, MA 01944 (US)
(74) Representative: FRKelly
(86) International application number: PCT/US2021/047778
(87) International publication number: WO 2022/047051

(56) References cited:
- WO-A1-2015/130728
- WO-A1-2018/197997
- WO-A1-2019/164794
- WO-A1-2020/109625
- WO-A1-2020/132471
- MCDONALD GABRIELLE ET AL: "Selective Vulnerability to Pyrimidine Starvation in Hematologic Malignancies Revealed by AG-636, a Novel Clinical-Stage Inhibitor of Dihydroorotate Dehydrogenase", MOLECULAR CANCER THERAPEUTICS, vol. 19, no. 12, 20 October 2020 (2020-10-20), US, pages 2502 - 2515, XP055841000, ISSN: 1535-7163, DOI: 10.1158/1535-7163.MCT-20-0550

## Description

### BACKGROUND

This application claims priority to U.S. Provisional Application No.: 63/070,984, filed August 27, 2020.

Lymphoma is a type of cancer that begins in cells of the lymph system. Lymphomas are classified generally as being either Hodgkin lymphoma or non-Hodgkin lymphoma. Hodgkin lymphomas are marked by the presence of specific white blood cells called Reed-Sternberg cells, and are generally more rare. Non-Hodgkin lymphomas are marked by the absence of Reed-Sternberg cells and account for nearly 90% of all lymphomas, including subtypes such as Burkitt's lymphoma, chronic lymphocytic leukemia (CLL), diffuse large B-cell lymphoma (DLBCL), cutaneous B-cell lymphoma (CBCL), cutaneous T-cell lymphoma (CTCL), and follicular lymphoma. According to the American Cancer Society, non-Hodgkin lymphoma is one of the most common cancers in the United States affecting about 1 in 42 men and 1 in 52 women.

R-CHOP is the name of a combination of cancer drugs that are used as a first line treatment for non-Hodgkin lymphoma, particularly DLBCL, which represent about 25% of all lymphomas. R-CHOP is made up of the drugs rituximab, cyclophosphamide, doxorubicin, vincristine, and prednisolone. Despite the aggressive course of DLBCL, about 50% to 70% of patients may be cured by standard treatment with R-CHOP. Nevertheless, R-CHOP, alone, is inadequate in 30% to 50% of patients. Among patients for whom R-CHOP therapy fails, 20% suffer from primary refractory disease (progress during or right after treatment) and 30% relapse after achieving complete remission.

WO2015130728 and WO2020109625 each disclose R-CHOP therapy for use in the treatment of lymphomas.

In view of the foregoing, there remains an unmet need for new therapeutic approaches that target lymphomas more effectively.

### SUMMARY

The dihydroorotate dehydrogenase inhibitor 1-methyl-5-(2'-methyl-[1,1'-biphenyl]-yl)-1H-benzo[d][1,2,3]triazole-7-carboxylic acid and its pharmaceutically acceptable salts thereof, also referred to as the "compound of Formula (I)" or "Compound 1" is useful in, among other things, the treatment of hematological malignancies, and has now been found to provide a therapeutic advantage when used in combination with CHOP based therapies.

Compound 1 is disclosed in WO2019164794, WO2020132471 and WO2018197997 for use on the treatment of lymphomas.

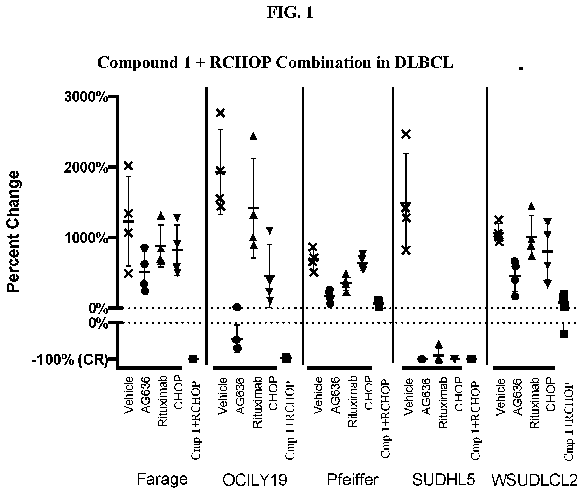

For example, complete tumor regression was observed in various mouse lymphoma models using the combination of Compound 1 and R-CHOP, whereas tumor growth continued in mice who were treated with CHOP or rituximab alone. See e.g., FIGs 1-3, which show complete regression of certain diffuse large B-cell lymphoma (DLBCL), mantle cell lymphoma (MCL), Burkitt lymphoma, Hodgkin lymphoma, and undifferentiated lymphoma mouse models following treatment with Compound 1 and R-CHOP.

In still another aspect, the application provides an anti-cancer agent for use in treating a lymphoma in combination with an anti-cancer therapy, wherein the anti-cancer agent is represented by Formula (I): 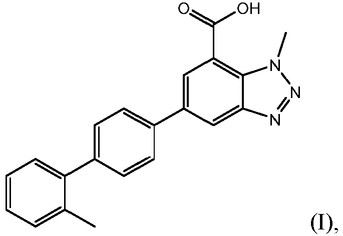 or a pharmaceutically acceptable salt thereof; and wherein the anti-cancer therapy comprises a combination of cyclophosphamide, doxorubicin or a pharmaceutically acceptable salt thereof, vincristine or a pharmaceutically acceptable salt thereof, and prednisolone. In one embodiment, the anti-cancer therapy further comprises rituximab. In one embodiment, the pharmaceutically acceptable salt thereof of Compound 1 is a tris(hydroxymethyl)aminomethane salt or a sodium salt. In one embodiment, the pharmaceutically acceptable salt thereof of doxorubicin is a hydrochloric acid salt. In one embodiment, the pharmaceutically acceptable salt thereof of vincristine is a sulfate salt.

Also described is a pharmaceutical composition comprising an anti-cancer agent and an anti-cancer therapy, wherein the pharmaceutical composition optionally and additionally comprises an excipient, wherein the anti-cancer agent is represented by Formula (I): 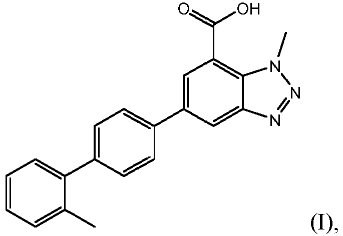 or a pharmaceutically acceptable salt thereof; and wherein the anti-cancer therapy comprises a combination of rituximab, cyclophosphamide, doxorubicin or a pharmaceutically acceptable salt thereof, vincristine or a pharmaceutically acceptable salt thereof, and prednisolone. In one embodiment, the pharmaceutically acceptable salt thereof of Compound 1 is a tris(hydroxymethyl)aminomethane salt or a sodium salt. In one embodiment, the pharmaceutically acceptable salt thereof of doxorubicin is a hydrochloric acid salt. In one embodiment, the pharmaceutically acceptable salt thereof of vincristine is a sulfate salt.

Other features or advantages will be apparent from the following detailed description of the drawings and several embodiments, and also from the appended claims.

### BRIEF DESCRIPTION OF THE DRAWINGS

**FIG. 1** shows percent change in tumour volume in DLBCL mouse models treated with Compound 1 (100 mg/kg BID), rituximab (20 mg/kg BIW), CHOP (C - 30 mg/kg QD x1 IP, D - 2.475 mg/kg QD x1 IV, V - 0.375 mg/kg QD x1 IV, P - 0.15 mg/kg QD x5 PO) and the combination of Compound 1 (100 mg/kg BID) with R-CHOP (R - 20 mg/kg BIW IP, C - 30 mg/kg QD x1 IP, D - 2.475 mg/kg QD x1 IV, V - 0.375 mg/kg QD x1 IV, P - 0.15 mg/kg QD x5 PO).

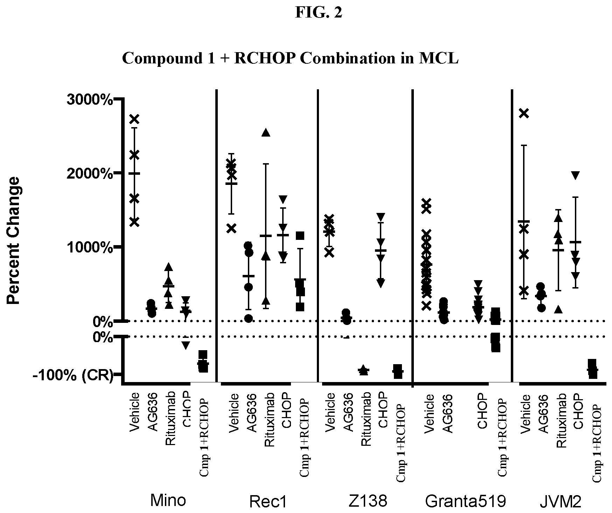

**FIG. 2** shows percent change in tumour volume in MCL mouse models treated with Compound 1 (100 mg/kg BID), rituximab (20 mg/kg BIW), CHOP (C - 30 mg/kg QD x1 IP, D - 2.475 mg/kg QD x1 IV, V - 0.375 mg/kg QD x1 IV, P - 0.15 mg/kg QD x5 PO) and the combination of Compound 1 (100 mg/kg BID) with R-CHOP (R - 20 mg/kg BIW IP, C - 30 mg/kg QD x1 IP, D - 2.475 mg/kg QD x1 IV, V - 0.375 mg/kg QD x1 IV, P - 0.15 mg/kg QD x5 PO).

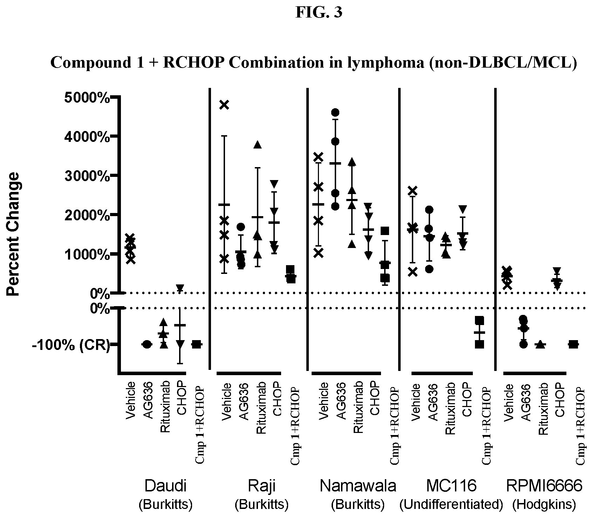

**FIG. 3** shows percent change in tumour volume in Burkitt, undifferentiated, and Hodgkin lymphoma mouse models treated with Compound 1 (100 mg/kg BID), rituximab (20 mg/kg BIW), CHOP (C - 30 mg/kg QD x1 IP, D - 2.475 mg/kg QD x1 IV, V - 0.375 mg/kg QD x1 IV, P - 0.15 mg/kg QD x1 IV) and the combination of Compound 1 (100 mg/kg BID) with R-CHOP (R - 20 mg/kg BIW IP, C - 30 mg/kg QD x1 IP, D - 2.475 mg/kg QD x1 IV, V - 0.375 mg/kg QD x5 PO, P - 0.15 mg/kg QD x5 PO).

### DETAILED DESCRIPTION

1-methyl-5-(2'-methyl-[1,1'-biphenyl]-yl)-1*H*-benzo[*d*][1,2,3]triazole-7-carboxylic acid, hereinafter also referred to as the "compound of Formula (I)" or "Compound 1" has the chemical structure shown below and is characterized as an inhibitor of DHODH. See *e.g.,* International Patent Application Publication Nos. WO 2014/128669 and WO 2019/164794 and US Patent No. 9,630,932.

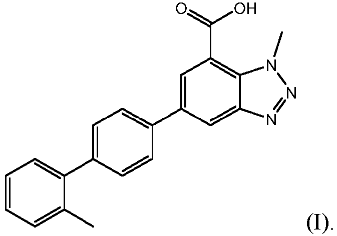

The compound of Formula (I) was developed to treat conditions and disorders that would benefit from inhibition of DHODH, such as but not limited to solid cancers and hematological cancers.

The invention is defined by the claims. In one aspect, the present invention is directed to a an anti-cancer agent represented by Formula (I) or a pharmaceutically acceptable salt thereof for use in treating lymphoma in combination with an anti-cancer therapy, wherein the anti-cancer therapy comprises a combination of rituximab, cyclophosphamide, doxorubicin or a pharmaceutically acceptable salt thereof, vincristine or a pharmaceutically acceptable salt thereof, and prednisolone, wherein the lymphoma is diffuse large B-cell lymphoma (DLBCL), mantle cell lymphoma (MCL), or Burkitt lymphoma. In one embodiment, the pharmaceutically acceptable salt thereof of Compound 1 is a tris(hydroxymethyl)aminomethane salt or a sodium salt. In one embodiment, the pharmaceutically acceptable salt thereof of doxorubicin is a hydrochloric acid salt. In one embodiment, the pharmaceutically acceptable salt thereof of vincristine is a sulfate salt.

The lymphoma is a B-cell lymphoma selected from follicular lymphoma, DLBCL, MCL, Burkitt, and lymphoplasmacytic lymphoma. In some embodiments, the non-Hodgkin lymphoma is a B-cell lymphoma selected from DLBCL, MCL, and Burkitt lymphoma. In some embodiments, the non-Hodgkin lymphoma is a B-cell lymphoma selected from DLBCL and MCL. In some embodiments, the non-Hodgkin lymphoma is the B-cell lymphoma DLBCL. In some embodiments, the non-Hodgkin lymphoma is the B-cell lymphoma MCL.

As used herein the terms "subject" and "patient" may be used interchangeably, and mean a mammal in need of treatment, *e.g.,* companion animals (*e.g.,* dogs, cats, and the like), farm animals (*e.g.,* cows, pigs, horses, sheep, goats and the like) and laboratory animals (*e.g.,* rats, mice, guinea pigs and the like). Typically, the subject is a human in need of treatment.

An "effective amount" of the compound of Formula (I), or a pharmaceutically acceptable salt, is an amount sufficient to provide a therapeutic benefit in the treatment of a lymphoma or to delay or minimize one or more symptoms associated with a lymphoma when combined with anti-cancer therapies, such as CHOP or R-CHOP. An "effective amount" of the anti-cancer therapy, as described herein, is an amount sufficient to provide a therapeutic benefit in the treatment of a condition (such as a lymphoma) or to delay or minimize one or more symptoms associated with the condition (such as a lymphoma) when combined with the compound of Formula (I), or a pharmaceutically acceptable salt thereof. The terms "therapeutically effective amount" and "effective amount" are used interchangeably. The term "effective amount" can encompass an amount that improves overall therapy, reduces or avoids symptoms, signs, or causes of a lymphoma, and/or enhances the therapeutic efficacy of another therapeutic agent. In certain embodiments, an effective amount is an amount sufficient for eliciting therapeutic effects in the treatment of a lymphoma.

The precise amount of the compound of Formula (I) (or pharmaceutically acceptable salt thereof), or the anti-cancer therapy administered to a subject will depend on various factors, such as the given drug, the pharmaceutical formulation, the route of administration, the type of disease or disorder, the identity of the subject being treated, and the like, but can nevertheless be routinely determined by one skilled in the art. For example, determination of an effective amount will also depend on the degree, severity and type of cell proliferation. The skilled artisan will be able to determine appropriate dosages depending on these and other factors. Suitable dosages are known for approved therapeutic agents and can be adjusted by the skilled artisan according to the condition of the subject, the type of condition(s) being treated and the amount of the compound of Formula (I) or pharmaceutically acceptable salt thereof. In cases where no amount is expressly noted, an effective amount should be assumed. Non-limiting examples of an effective amount of the compound of Formula (I) or pharmaceutically acceptable salt thereof are provided herein below.

An effective amount of the compound of Formula (I), or a pharmaceutically acceptable salt thereof, is generally in the range from 0.1 µg to 100 mg/kg of body weight of the recipient (mammal) per day and particularly typically in the range from 1 to 10 mg/kg of body weight per day. Thus, the actual amount per day for an adult mammal weighing 70 kg is usually between 70 and 700 mg, where this amount can be administered as an individual dose per day or usually in a series of part-doses (such as, for example, two, three, four, five or six) per day, so that the total daily dose is the same. The effective dose of the compound of Formula (I) or pharmaceutically acceptable salt thereof to a subject can be 10 µg -500 mg. Effective amounts of the anti-cancer therapies CHOP and R-CHOP are known to those skilled in the art. Exemplary amounts are further discussed below.

The term "treat" means decrease, suppress, attenuate, diminish, arrest, or stabilize the development or progression of a lymphoma, lessen the severity of the lymphoma or improve the symptoms associated with the lymphoma. Treatment may also be continued after symptoms have resolved, for example to reduce the likelihood of or delay their recurrence.

Positive therapeutic effects in lymphoma can be measured in a number of ways. The administration of a therapeutically effective amount of the combinations herein described are advantageous over the individual component compounds. As used herein "advantageous combinations" are those combinations that provide at least one of the following improved properties when compared to the individual administration of a therapeutically effective amount of a component compound: i) a greater anticancer effect than the most active single agent, alone; ii) synergistic anticancer effect; or iii) additive activity.

The term "relapsed" refers to a situation where, after therapy, patients who have had a remission of lymphoma, have a return of cancer cells.

The term "refractory or resistant" refers to a circumstance where patients, even after intensive treatment, have residual cancer cells in their body.

As used herein, "double hit diffuse large B cell lymphoma" refers to a form of lymphoma or diffuse large B cell lymphoma where the lymphoma cells are altered at two oncogenes which are *c-MYC* and *BCL2* or *BCL6.* In one embodiment, double hit diffuse large B cell lymphoma is treated in the method, and is characterized by gene alterations at *c-MYC* and *BCL2.* In another embodiment, double hit diffuse large B cell lymphoma is treated in the method, and is characterized by gene alterations at *c-MYC* and *BCL6.*

As used herein, "triple hit diffuse large B cell lymphoma" refers to a form of lymphoma or diffuse large B cell lymphoma where the lymphoma cells are altered at three oncogenes which are *c-MYC, BCL2* and *BCL6.* In one embodiment, triple hit diffuse large B cell lymphoma is treated in the method.

In this description, a "pharmaceutically acceptable salt" is a pharmaceutically acceptable, organic or inorganic acid or base salt of a compound described herein. Representative pharmaceutically acceptable, organic or inorganic base salts include, *e.g.,* alkali metal salts *(e.g.,* sodium), alkaline earth metal salts (*e.g.,* calcium), and ammonium salts (*e.g.,* tris(hydroxymethyl)aminomethane, hydrabamine, and N-methylglucamine ammonium salt). Representative pharmaceutically acceptable, organic or inorganic acid or base salts include, *e.g.,* the acetate, amsonate (4,4-diaminostilbene-2, 2 -disulfonate), benzene sulfonate, benzonate, bicarbonate, bisulfate, bitartrate, borate, bromide, butyrate, calcium edetate, camsylate, carbonate, chloride, citrate, clavulariate, dihydrochloride, edetate, edisylate, estolate, esylate, fiunarate, gluceptate, gluconate, glutamate, glycollylarsanilate, hexafluorophosphate, hexylresorcinate, hydrobromide, hydrochloride, hydroxynaphthoate, iodide, isothionate, lactate, lactobionate, laurate, malate, maleate, mandelate, mesylate, methylbromide, methylnitrate, methyl sulfate, mucate, napsylate, nitrate, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosaliculate, suramate, tannate, tartrate, teoclate, tosylate, triethiodide, and valerate salts. A pharmaceutically acceptable salt can have more than one charged atom in its structure. In this instance the pharmaceutically acceptable salt can have multiple counterions. Thus, a pharmaceutically acceptable salt can have one or more charged atoms and/or one or more counterions.

In one embodiment, the methods disclsoed herein comprise administration or co-administration of the anti-cancer therapy CHOP or R-CHOP. The term "co-administering" as used herein with respect to the anti-cancer therapy means that the anti-cancer therapy may be administered together with Compound 1, or a pharmaceutically acceptable salt thereof, as part of a single dosage form (such as a composition comprising Compound 1, or a pharmaceutically acceptable salt thereof, and the anti-cancer therapy) or as separate, multiple dosage forms. Alternatively, the anti-cancer therapy may be administered prior to, consecutively with, or following the administration of Compound 1, or a pharmaceutically acceptable salt thereof. In such combination therapy treatment, both Compound 1, or a pharmaceutically acceptable salt thereof, and the anti-cancer therapy are administered by conventional methods. The administration of a composition comprising both Compound 1, or a pharmaceutically acceptable salt thereof, and the anti-cancer therapy, to a subject does not preclude the separate administration of that same therapeutic agent, any other second therapeutic agent or any compound provided herein to said subject at another time during a course of treatment. The term "co-administering" as used herein with respect to an additional cancer treatment means that the additional cancer treatment may occur prior to, consecutively with, concurrently with or following the administration of a compound provided herein.

The anti-cancer therapy is R-CHOP (i.e., a combination of rituximab, cyclophosphamide, doxorubicin or a pharmaceutically acceptable salt thereof such as a hydrochloric acid salt, vincristine or a pharmaceutically acceptable salt thereof such as a sulfate salt, and prednisolone).

Depending on the lymphoma to be treated and the subject's condition, the cyclophosphamide of the anti-cancer therapy described herein, may be administered by oral, parenteral (e.g., intramuscular, intraperitoneal, intravenous, CIV (continuous intravenous), intracisternal injection or infusion, subcutaneous injection, or implant), inhalation, nasal, vaginal, rectal, sublingual, or topical (e.g., transdermal or local) routes of administration. Cyclophosphamide may be formulated, alone or together with Compound 1 and/or one or more active agent(s), in suitable dosage unit with pharmaceutically acceptable excipients, carriers, adjuvants and vehicles, appropriate for each route of administration.

In one embodiment, cyclophosphamide is administered by, e.g., intravenous (IV) or oral routes.

In certain embodiments, treatment cycles comprise multiple doses of cyclophosphamide administered to a subject in need thereof over multiple days (*e.g.,* 1, 2, 3, 4, 5, 6, 7, 8, 9, 10, 11, 12, 13, 14, or greater than 14 days). In certain embodiments, treatment cycles comprise multiple doses of cyclophosphamide administered to a subject in need thereof over multiple weeks (e.g., 1, 2, 3, 4, or greater than 5 weeks). Suitable dosage amounts for the methods disclosed herein include, e.g., therapeutically effective amounts and prophylactically effective amounts. For example, in certain embodiments, the amount of cyclophosphamide administered by the present methods may range, e.g., from about 100 mg/m² to about 1500 mg/m², from about 200 mg/m² to about 1500 mg/m², from about 300 mg/m² to about 1500 mg/m², from about 400 mg/m² to about 1500 mg/m², from about 450 mg/m² to about 1500 mg/m², from about 500 mg/m² to about 1500 mg/m², from about 550 mg/m² to about 1500 mg/m², from about 600 mg/m² to about 1500 mg/m², from about 650 mg/m² to about 1500 mg/m², from about 700 mg/m² to about 1500 mg/m², from about 750 mg/m² to about 1500 mg/m², from about 100 mg/m² to about 1250 mg/m², from about 100 mg/m² to about 1150 mg/m², from about 100 mg/m² to about 1000 mg/m², from about 100 mg/m² to about 900 mg/m², from about 100 mg/m² to about 850 mg/m², from about 100 mg/m² to about 800 mg/m², from about 100 mg/m² to about 750 mg/m², from about 200 mg/m² to about 1400 mg/m², from about 300 mg/m² to about 1300 mg/m², from about 400 mg/m² to about 1200 mg/m², from about 500 mg/m² to about 1100 mg/m², from about 600 mg/m² to about 1000 mg/m², from about 600 mg/m² to about 900 mg/m², from about 600 mg/m² to about 800 mg/m², from about 700 mg/m² to about 800 mg/m², or from about 725 mg/m² to about 775 mg/m². In certain embodiments, the amount of cyclophosphamide administered by the present methods is about 500 mg/m², about 550 mg/m², about 600 mg/m², about 650 mg/m², about 700 mg/m², about 750 mg/m², about 800 mg/m², or about 850 mg/m². In certain embodiments, the amount of cyclophosphamide administered by the present methods is about 750 mg/m².

In certain embodiments, the amount of cyclophosphamide is from about 1 mg/kg to about 100 mg/kg, from about 5 mg/kg to about 100 mg/kg, from about 10 mg/kg to about 100 mg/kg, from about 15 mg/kg to about 100 mg/kg, from about 20 mg/kg to about 100 mg/kg, from about 25 mg/kg to about 100 mg/kg, from about 30 mg/kg to about 100 mg/kg, from about 35 mg/kg to about 100 mg/kg, from about 1 mg/kg to about 90 mg/kg, from about 1 mg/kg to about 80 mg/kg, from about 1 mg/kg to about 70 mg/kg, from about 1 mg/kg to about 60 mg/kg, from about 1 mg/kg to about 50 mg/kg, from about 1 mg/kg to about 40 mg/kg, from about 1 mg/kg to about 35 mg/kg, from about 1 mg/kg to about 30 mg/kg, from about 1 mg/kg to about 100 mg/kg, from about 10 mg/kg to about 40 mg/kg, from about 15 mg/kg to about 40 mg/kg, from about 20 mg/kg to about 40 mg/kg, or from about 25 mg/kg to about 35 mg/kg. In certain embodiments, the amount of cyclophosphamide is about 10 mg/kg, 15 mg/kg, 20 mg/kg, 25 mg/kg, 30 mg/kg, 35 mg/kg, or 40 mg/kg. In certain embodiments, the amount of cyclophosphamide is about 30 mg/kg. In certain embodiments, the cyclophosphamide is administered every day (QD) at one or more of the dosages recited herein. In certain embodiments, the cyclophosphamide is administered every day (QD) at one or more of the dosages recited herein on certain days of treatment (e.g., Days 1 and 22).

Depending on the lymphoma to be treated and the subject's condition, the doxorubicin (e.g., doxorubicin hydrochloride) of the anti-cancer therapy described herein may be administered by oral, parenteral (*e.g.,* intramuscular, intraperitoneal, intravenous, CIV, intracisternal injection or infusion, subcutaneous injection, or implant), inhalation, nasal, vaginal, rectal, sublingual, or topical (*e.g.,* transdermal or local) routes of administration. Doxorubicin or doxorubicin hydrochloride may be formulated, alone or together with Compound 1 and/or one or more active agent(s), in suitable dosage unit with pharmaceutically acceptable excipients, carriers, adjuvants and vehicles, appropriate for each route of administration.

In one embodiment, doxorubicin (e.g., doxorubicin hydrochloride) is administered by, *e.g.,* intravenous (IV) routes.

In certain embodiments, treatment cycles comprise multiple doses of doxorubicin (e.g., doxorubicin hydrochloride) administered to a subject in need thereof over multiple days (*e.g.,* 1, 2, 3, 4, 5, 6, 7, 8, 9, 10, 11, 12, 13, 14, or greater than 14 days). In certain embodiments, treatment cycles comprise multiple doses of doxorubicin (e.g., doxorubicin hydrochloride) administered to a subject in need thereof over multiple weeks (*e.g.,* 1, 2, 3, 4, or greater than 5 weeks). Suitable dosage amounts for the methods provided herein include, e.g., therapeutically effective amounts and prophylactically effective amounts. For example, in certain embodiments, the amount of doxorubicin (e.g., doxorubicin hydrochloride) administered by the present methods is from about 1 mg/m² to about 200 mg/m², from about 1 mg/m² to about 150 mg/m², from about 1 mg/m² to about 100 mg/m², from about 10 mg/m² to about 100 mg/m², from about 15 mg/m² to about 100 mg/m², from about 20 mg/m² to about 100 mg/m², from about 25 mg/m² to about 100 mg/m², from about 30 mg/m² to about 100 mg/m², from about 35 mg/m² to about 100 mg/m², from about 40 mg/m² to about 100 mg/m², from about 45 mg/m² to about 100 mg/m², from about 50 mg/m² to about 100 mg/m², from about 1 mg/m² to about 95 mg/m², from about 1 mg/m² to about 90 mg/m², from about 1 mg/m² to about 85 mg/m², from about 1 mg/m² to about 80 mg/m², from about 1 mg/m² to about 75 mg/m², from about 1 mg/m² to about 70 mg/m², from about 1 mg/m² to about 65 mg/m², from about 1 mg/m² to about 60 mg/m², from about 1 mg/m² to about 55 mg/m², or from about 1 mg/m² to about 50 mg/m². In certain embodiments, the amount of doxorubicin (e.g., doxorubicin hydrochloride) administered by the present methods is about 25 mg/m², about 30 mg/m², about 35 mg/m², about 40 mg/m², about 45 mg/m², about 50 mg/m², about 55 mg/m², about 60 mg/m², or about 65 mg/m². In certain embodiments, the amount of doxorubicin (e.g., doxorubicin hydrochloride) administered by the present methods is about 50 mg/m².

In certain embodiments, the amount of doxorubicin (e.g., doxorubicin hydrochloride) administered by the present methods is from about 0.5 mg/kg to about 5 mg/kg, from about 1 mg/kg to about 5 mg/kg, from about 1.25 mg/kg to about 5 mg/kg, from about 1.5 mg/kg to about 5 mg/kg, from about 1.75 mg/kg to about 5 mg/kg, from about 2 mg/kg to about 5 mg/kg, from about 2.25 mg/kg to about 5 mg/kg, from about 2.5 mg/kg to about 5 mg/kg, from about 0.5 mg/kg to about 4 mg/kg, from about 0.5 mg/kg to about 3.5 mg/kg, from about 0.5 mg/kg to about 3.25 mg/kg, from about 0.5 mg/kg to about 3.0 mg/kg, from about 0.5 mg/kg to about 2.75 mg/kg, from about 0.5 mg/kg to about 2.5 mg/kg, from about 2 mg/kg to about 3 mg/kg, or from about 2.25 mg/kg to about 2.5 mg/kg. In certain embodiments, the amount of doxorubicin (e.g., doxorubicin hydrochloride) administered by the present methods is about 2.475 mg/kg. In certain embodiments, the doxorubicin (e.g., doxorubicin hydrochloride) is administered every day (QD) at one or more of the dosages recited herein. In certain embodiments, the doxorubicin (e.g., doxorubicin hydrochloride) is administered every day (QD) at one or more of the dosages recited herein on certain days of treatment (e.g., Days 1 and 22).

Depending on the lymphoma to be treated and the subject's condition, the vincristine (e.g., vincristine sulfate) of the anti-cancer therapy described herein may be administered by oral, parenteral (*e.g.,* intramuscular, intraperitoneal, intravenous, CIV, intracisternal injection or infusion, subcutaneous injection, or implant), inhalation, nasal, vaginal, rectal, sublingual, or topical (*e.g.,* transdermal or local) routes of administration. Vincristine (e.g., vincristine sulfate) may be formulated, alone or together with Compound 1 and/or one or more active agent(s), in suitable dosage unit with pharmaceutically acceptable excipients, carriers, adjuvants and vehicles, appropriate for each route of administration.

In one embodiment, vincristine (e.g., vincristine sulfate) is administered by, *e.g.,* intravenous (IV) routes.

In certain embodiments, treatment cycles comprise multiple doses of vincristine (e.g., vincristine sulfate) administered to a subject in need thereof over multiple days (*e.g.,* 1, 2, 3, 4, 5, 6, 7, 8, 9, 10, 11, 12, 13, 14, or greater than 14 days). In certain embodiments, treatment cycles comprise multiple doses of vincristine (e.g., vincristine sulfate) administered to a subject in need thereof over multiple weeks (*e.g.,* 1, 2, 3, 4, or greater than 5 weeks). Suitable dosage amounts for the methods provided herein include, *e.g.,* therapeutically effective amounts and prophylactically effective amounts. For example, in certain embodiments, the amount of vincristine (e.g., vincristine sulfate) administered by the present methods is from about 0.2 mg/m² to about 10 mg/m², from about 0.2 mg/m² to about 5 mg/m², from about 0.4 mg/m² to about 5 mg/m², from about 0.6 mg/m² to about 5 mg/m², from about 0.8 mg/m² to about 5 mg/m², from about 1 mg/m² to about 5 mg/m², from about 1.2 mg/m² to about 5 mg/m², from about 1.4 mg/m² to about 5 mg/m², from about 0.2 mg/m² to about 3 mg/m², from about 0.2 mg/m² to about 2.5 mg/m², from about 0.2 mg/m² to about 2.2 mg/m², from about 0.2 mg/m² to about 2.0 mg/m², from about 0.2 mg/m² to about 1.8 mg/m², from about 1.6 mg/m² to about 1.4 mg/m², from about 0.4 mg/m² to about 2.5 mg/m², from 0.6 mg/m² to about 2.2 mg/m², from 0.8 mg/m² to about 2.0 mg/m², from 0.8 mg/m² to about 2.0 mg/m², from 1 mg/m² to about 1.8 mg/m², or from 1.2 mg/m² to about 1.6 mg/m². In certain embodiments, the amount of vincristine (e.g., vincristine sulfate) administered by the present methods is about 1 mg/m², 1.2 mg/m², about 1.4 mg/m², 1.4 mg/m², 1.6 mg/m², 1.8 mg/m², or 2 mg/m². In certain embodiments, the amount of vincristine (e.g., vincristine sulfate) administered by the present methods is about 1.4 mg/m².

In certain embodiments, the amount of vincristine (e.g., vincristine sulfate) administered by the present methods is from about 0.1 mg/kg to about 1 mg/kg, from about 0.2 mg/kg to about 1 mg/kg, from about 0.25 mg/kg to about 1 mg/kg, from about 0.275 mg/kg to about 1 mg/kg, from about 0.3 mg/kg to about 1 mg/kg, from about 0.325 mg/kg to about 1 mg/kg, from about 0.35 mg/kg to about 1 mg/kg, from about 0.375 mg/kg to about 1 mg/kg, from about 0.4 mg/kg to about 1 mg/kg, from about 0.1 mg/kg to about 0.5 mg/kg, from about 0.1 mg/kg to about 0.475 mg/kg, from about 0.1 mg/kg to about 0.45 mg/kg, from about 0.1 mg/kg to about 0.425 mg/kg, from about 0.1 mg/kg to about 4 mg/kg, from about 0.1 mg/kg to about 0.375 mg/kg, from about 0.1 mg/kg to about 0.35 mg/kg, from about 0.3 mg/kg to about 0.4 mg/kg, from about 0.325 mg/kg to about 0.4 mg/kg, or from about 0.35 mg/kg to about 0.4 mg/kg. In certain embodiments, the amount of vincristine (e.g., vincristine sulfate) administered by the present methods is about 0.375 mg/kg.

Depending on the lymphoma to be treated and the subject's condition, the prednisone of the anti-cancer therapy described herein may be administered by oral, parenteral (*e.g.,* intramuscular, intraperitoneal, intravenous, CIV, intracisternal injection or infusion, subcutaneous injection, or implant), inhalation, nasal, vaginal, rectal, sublingual, or topical (*e.g.,* transdermal or local) routes of administration. Prednisone may be formulated, alone or together with Compound 1 and/or one or more active agent(s), in suitable dosage unit with pharmaceutically acceptable excipients, carriers, adjuvants and vehicles, appropriate for each route of administration.

In one embodiment, prednisone is administered by, *e.g.,* intravenous (IV) or oral routes.

In certain embodiments, treatment cycles comprise multiple doses of prednisone administered to a subject in need thereof over multiple days (*e.g.,* 1, 2, 3, 4, 5, 6, 7, 8, 9, 10, 11, 12, 13, 14, or greater than 14 days). In certain embodiments, treatment cycles comprise multiple doses of prednisone administered to a subject in need thereof over multiple weeks (*e.g.,* 1, 2, 3, 4, or greater than 5 weeks). Suitable dosage amounts for the methods provided herein include, *e.g.,* therapeutically effective amounts and prophylactically effective amounts. For example, in certain embodiments, the amount of prednisone administered by the present methods is from about 25 mg/m² to about 200 mg/m², from about 50 mg/m² to about 200 mg/m², from about 75 mg/m² to about 200 mg/m², from about 100 mg/m² to about 200 mg/m², from about 25 mg/m² to about 175 mg/m², from about 125 mg/m² to about 150 mg/m², from about 25 mg/m² to about 125 mg/m², from about 25 mg/m² to about 100 mg/m², from about 50 mg/m² to about 175 mg/m², from about 75 mg/m² to about 150 mg/m², or from about 75 mg/m² to about 125 mg/m².

In certain embodiments, the amount of prednisone administered by the present methods is about 25 mg/m², about 50 mg/m², about 75 mg/m², about 100 mg/m², about 125 mg/m², or about 150 mg/m². In certain embodiments, the amount of prednisone is about 100 mg/m².

In certain embodiments, the amount of prednisone administered by the present methods is from about 25 mg to about 200 mg, from about 50 mg to about 200 mg, from about 75 mg to about 200 mg, from about 100 mg to about 200 mg, from about 25 mg to about 175 mg, from about 125 mg to about 150 mg, from about 25 mg to about 125 mg, from about 25 mg to about 100 mg, from about 50 mg to about 175 mg, from about 75 mg to about 150 mg, or from about 75 mg to about 125 mg.

In certain embodiments, the amount of prednisone administered by the present methods is about 25 mg, about 50 mg, about 75 mg, about 100 mg, about 125 mg, or about 150 mg. In certain embodiments, the amount of prednisone is about 100 mg.

In certain embodiments, the amount of prednisone administered by the present methods is from about 0.05 mg/kg to about 0.3 mg/kg, from about 0.075 mg/kg to about 0.3 mg/kg, from about 0.1 mg/kg to about 0.3 mg/kg, from about 0.125 mg/kg to about 0.3 mg/kg, from about 0.15 mg/kg to about 0.3 mg/kg, from about 0.175 mg/kg to about 0.3 mg/kg, from about 0.05 mg/kg to about 0.275 mg/kg, from about 0.05 mg/kg to about 0.25 mg/kg, from about 0.05 mg/kg to about 0.2 mg/kg, from about 0.05 mg/kg to about 0.175 mg/kg, from about 0.05 mg/kg to about 0.15 mg/kg, from about 0.01 mg/kg to about 0.2 mg/kg, or from about 0.125 mg/kg to about 0.175 mg/kg. In certain embodiments, the amount of prednisone administered by the present methods is about 0.15 mg/kg. In certain embodiments, the prednisone is administered every day (QD) at one or more of the dosages recited herein. In certain embodiments, the prednisone is administered every day (QD) at one or more of the dosages recited herein on certain days of treatment (e.g., Days 1-5 and Day 22-27).

Depending on the lymphoma to be treated and the subject's condition, the rituximab of the anti-cancer therapy described herein may be administered by oral, parenteral (*e.g.,* intramuscular, intraperitoneal, intravenous, CIV, intracisternal injection or infusion, subcutaneous injection, or implant), inhalation, nasal, vaginal, rectal, sublingual, or topical (*e.g.,* transdermal or local) routes of administration. Rituximab may be formulated, alone or together with Compound 1 and/or one or more active agent(s), in suitable dosage unit with pharmaceutically acceptable excipients, carriers, adjuvants and vehicles, appropriate for each route of administration.

In one embodiment, rituximab is administered by, *e.g.,* intravenous (IV) route.

In certain embodiments, treatment cycles comprise multiple doses of rituximab administered to a subject in need thereof over multiple days (*e.g.,* 1, 2, 3, 4, 5, 6, 7, 8, 9, 10, 11, 12, 13, 14, or greater than 14 days). In certain embodiments, treatment cycles comprise multiple doses of rituximab administered to a subject in need thereof over multiple weeks (*e.g.,* 1, 2, 3, 4, or greater than 5 weeks). Suitable dosage amounts for the methods provided herein include, e.g., therapeutically effective amounts and prophylactically effective amounts. For example, in certain embodiments, the amount of rituximab is from about 200 mg/m² to about 500 mg/m², from about 225 mg/m² to about 500 mg/m², from about 250 mg/m² to about 500 mg/m², from about 275 mg/m² to about 500 mg/m², from about 300 mg/m² to about 500 mg/m², from about 325 mg/m² to about 500 mg/m², from about 350 mg/m² to about 500 mg/m², from about 375 mg/m² to about 500 mg/m², from about 200 mg/m² to about 475 mg/m², from about 200 mg/m² to about 450 mg/m², from about 200 mg/m² to about 425 mg/m², from about 200 mg/m² to about 400 mg/m², from about 200 mg/m² to about 375 mg/m², from about 225 mg/m² to about 475 mg/m², from about 250 mg/m² to about 450 mg/m², from about 275 mg/m² to about 425 mg/m², from about 300 mg/m² to about 400 mg/m², from about 325 mg/m² to about 400 mg/m², or from about 350 mg/m² to about 400 mg/m². In certain embodiments, the amount of rituximab is about 250 mg/m², 275 mg/m², 300 mg/m², 325 mg/m², 350 mg/m², 375 mg/m², 400 mg/m², or 425 mg/m². In certain embodiments, the amount of rituximab is about 375 mg/m².

In certain embodiments, the amount of rituximab is from about 1 mg/kg to about 100 mg/kg, from about 5 mg/kg to about 100 mg/kg, from about 10 mg/kg to about 100 mg/kg, from about 15 mg/kg to about 100 mg/kg, from about 20 mg/kg to about 100 mg/kg, from about 25 mg/kg to about 100 mg/kg, from about 30 mg/kg to about 100 mg/kg, from about 1 mg/kg to about 90 mg/kg, from about 1 mg/kg to about 80 mg/kg, from about 1 mg/kg to about 70 mg/kg, from about 1 mg/kg to about 60 mg/kg, from about 1 mg/kg to about 50 mg/kg, from about 1 mg/kg to about 40 mg/kg, from about 1 mg/kg to about 30 mg/kg, from about 1 mg/kg to about 20 mg/kg, from about 1 mg/kg to about 100 mg/kg, from about 5 mg/kg to about 30 mg/kg, from about 10 mg/kg to about 30 mg/kg, from about 10 mg/kg to about 25 mg/kg, or from about 15 mg/kg to about 25 mg/kg. In certain embodiments, the amount of rituximab is about 5 mg/kg, 10 mg/kg, 15 mg/kg, 20 mg/kg, 25 mg/kg, or 30 mg/kg. In certain embodiments, the amount of rituximab is about 20 mg/kg.

In certain embodiments, the rituximab is administered twice a week (BIW) at one or more of the dosages recited herein.

Depending on the lymphoma to be treated and the subject's condition, the second anti-cancer therapy comprises the administration of from about 250 mg/m² to about 450 mg/m² rituximab, from about 650 mg/m² to about 850 mg/m² cyclophosphamide, from about 1 mg/m² to about 100 mg/m² doxorubicin or doxorubicin hydrochloride, from about 0.5 mg/m² to about 3 mg/m² vincristine or vincristine sulfate, and from about 10 mg to about 200 mg or from about 10 mg/m² to about 200 mg/m² prednisone. For example, in some embodiments, the anti-cancer therapy of the present methods comprise the administration of about 375 mg/m² rituximab, about 750 mg/m² cyclophosphamide, about 50 mg/m² doxorubicin or doxorubicin hydrochloride, about 1.4 mg/m² vincristine or vincristine sulfate, and 100 mg or 100 mg/m² prednisone. In some embodiments, the rituximab, cyclophosphamide, doxorubicin (e.g., doxorubicin hydrochloride), and vincristine (vincristine sulfate), are administered intravenously.

Depending on the lymphoma to be treated and the subject's condition, the second anti-cancer therapy comprises the administration of from about 10 mg/kg to about 30 mg/kg rituximab, from about 20 mg/kg to about 40 mg/kg cyclophosphamide, from about 2 mg/kg to about 3 mg/kg doxorubicin or doxorubicin hydrochloride, from about 0.3 mg/kg to about 0.4 mg/kg vincristine or vincristine sulfate, and from about 0.1 mg/kg to about 0.2 mg/kg prednisone. For example, in some embodiments, the anti-cancer therapy of the present methods comprise the administration of about 20 mg/kg rituximab, about 30 mg/kg cyclophosphamide, about 2.475 mg/kg doxorubicin or doxorubicin hydrochloride, about 0.375 mg/kg vincristine or vincristine sulfate, and 0.15 mg/kg prednisone. In some embodiments, the rituximab, cyclophosphamide, doxorubicin (e.g., doxorubicin hydrochloride), and vincristine (vincristine sulfate), are administered intravenously.

In one embodiment, Compound 1 or a pharmaceutically acceptable salt thereof and the anti-cancer therapy can be administered simultaneously in the same or different formulations. In an alternative embodiment, Compound 1 or a pharmaceutically acceptable salt thereof and the anti-cancer therapy can be administered sequentially, i.e., at different points in time.

In some embodiments, the anti-cancer agent is a pharmaceutically acceptable salt of the compound of Formula (I). In one embodiment, the anti-cancer agent is the tris(hydroxymethyl)aminomethane ("Tris") salt of the compound of Formula (I). In another embodiment, the anti-cancer agent is the sodium salt of the compound of Formula (I).

In some embodiments, the compound of Formula (I) or a pharmaceutically acceptable salt thereof is a solid state or crystalline form of the compound of Formula (I), as described in PCT/US2019/067897 (WO 2020/132471), filed December 20, 2019. In one embodiment, the compound of Formula (I) is Form A, B, C, or D of the crystalline form of the compound of Formula (I). In one embodiment, the compound of Formula (I) is Form A, B, or C of the crystalline form of the Tris salt of the compound of Formula (I). In one embodiment, the compound of Formula (I) or the Tris salt thereof is in amorphous form.

In one embodiment, the dosing regimen for the lymphoma treatment method described in the foregoing paragraphs comprises administering an effective amount of the compound of Formula (I) or a pharmaceutically acceptable salt thereof (*e.g.,* the Tris salt of the compound of Formula (I) and the sodium salt of the compound of Formula (I)) once a week in one or more 28-day or 4-week cycles.

In one embodiment, the dosing regimen for the lymphoma treatment method described in the foregoing paragraphs comprises administering an effective amount of the compound of Formula (I) or a pharmaceutically acceptable salt thereof (*e.g.,* the Tris salt of the compound of Formula (I) and the sodium salt of the compound of Formula (I)) for a period of 2 to 5 days each week, with 1 cycle of therapy defined as 4 consecutive weeks of treatment

In one embodiment, the dosing regimen for the lymphoma treatment method described in the foregoing paragraphs comprises administering an effective amount of the compound of Formula (I) or a pharmaceutically acceptable salt thereof (*e.g.,* the Tris salt of the compound of Formula (I) and the sodium salt of the compound of Formula (I)) for a period of 2 to 5 consecutive days followed by a 2 to 5 day break each week, in one or more 28-day or 4-week cycle.

In one embodiment, the dosing regimen for the lymphoma treatment method described in the foregoing paragraphs comprises administering an effective amount of the compound of Formula (I) or a pharmaceutically acceptable salt thereof (*e.g.,* the Tris salt of the compound of Formula (I) and the sodium salt of the compound of Formula (I)) for 2 consecutive days followed by a 5-day break each week, in one or more 28-day or 4-week cycles.

In one embodiment, the dosing regimen for the lymphoma treatment method described in the foregoing paragraphs comprises administering an effective amount of the compound of Formula (I) or a pharmaceutically acceptable salt thereof (*e.g.,* the Tris salt of the compound of Formula (I) and the sodium salt of the compound of Formula (I)) for 3 consecutive days followed by a 4-day break each week, in one or more 28-day or 4-week cycles.

In one embodiment, the dosing regimen for the lymphoma treatment method described in the foregoing paragraphs comprises administering an effective amount of the compound of Formula (I), or a pharmaceutically acceptable salt thereof (*e.g.,* the Tris salt of the compound of Formula (I) and the sodium salt of the compound of Formula (I)) for 4 consecutive days followed by a 3-day break each week, in one or more 28-day or 4-week cycles.

In one embodiment, the dosing regimen for the lymphoma treatment method described in the foregoing paragraphs comprises administering an effective amount of the compound of Formula (I), or a pharmaceutically acceptable salt thereof (*e.g.,* the Tris salt of the compound of Formula (I) and the sodium salt of the compound of Formula (I)) for 5 consecutive days followed by a 2-day break each week, in one or more 28-day or 4-week cycles.

In one embodiment, the dosing regimen for the lymphoma treatment method described in the foregoing paragraphs comprises administering an effective amount of the compound of Formula (I), or a pharmaceutically acceptable salt thereof (*e.g.,* the Tris salt of the compound of Formula (I) and the sodium salt of the compound of Formula (I)) for a period of 2 to 5 consecutive days followed by a 2 to 5 day break each week, in one or more 28-day or 4-week cycle, where the number of consecutive days of treatment is increased in at least one of the weeks of the 28-day or 4-week cycle, in one or more 28-day or 4-week cycles.

In another embodiment, the dosing regimen for the lymphoma treatment method described in the foregoing paragraphs comprises administering an effective amount of the compound of Formula (I), or a pharmaceutically acceptable salt thereof (*e.g.,* the Tris salt of the compound of Formula (I) and the sodium salt of the compound of Formula (I)) for a period of 2 to 5 consecutive days followed by a 2 to 5 day break each week, in one or more 28-day or 4-week cycle, where the number of consecutive days of treatment is decreased in at least one of the weeks of the 28-day or 4-week cycle, in one or more 28-day or 4-week cycles.

In another embodiment, the effective amount of the compound of Formula (I), or a pharmaceutically acceptable salt thereof (*e.g.*, the Tris salt of the compound of Formula (I) and the sodium salt of the compound of Formula (I)) is administered to the subject for 2 consecutive days followed by a 5-day break for the first week of a 28-day or 4-week cycle, where the number of consecutive days of treatment is increased in at least one of the weeks of the 28-day or 4-week cycle.

In another embodiment, the effective amount of the compound of Formula (I), or a pharmaceutically acceptable salt thereof (*e.g.*. the Tris salt of the compound of Formula (I) and the sodium salt of the compound of Formula (I)) is administered to the subject for 2 consecutive days followed by a 5-day break for the first week of a 28-day or 4-week cycle, where the number of consecutive days of treatment is decreased in at least one of the weeks of a 28-day or 4-week cycle.

In another embodiment, the effective amount of the compound of Formula (I), or a pharmaceutically acceptable salt (*e.g.,* the Tris salt of the compound of Formula (I) and the sodium salt of the compound of Formula (I)) is administered to the subject for 3 consecutive days followed by a 4-day break for the first week of a 28-day or 4-week cycle, where the number of consecutive days of treatment is increased in at least one of the weeks of a 28-day or 4-week cycle.

In another embodiment, the effective amount of the compound of Formula (I), or a pharmaceutically acceptable salt thereof (*e.g.*. the Tris salt of the compound of Formula (I) and the sodium salt of the compound of Formula (I)) is administered to the subject for 3 consecutive days followed by a 4-day break for the first week of a 28-day or 4-week cycle, where the number of consecutive days of treatment is decreased in at least one of the weeks of a 28-day or 4-week cycle.

In another embodiment, the effective amount of the compound of Formula (I), or a pharmaceutically acceptable salt thereof (*e.g.*. the Tris salt of the compound of Formula (I) and the sodium salt of the compound of Formula (I)) is administered to the subject for 4 consecutive days followed by a 3-day break for the first week of a 28-day or 4-week cycle, where the number of consecutive days of treatment is increased in at least one of the weeks of the 28-day or 4-week cycle.

In another embodiment, the effective amount of the compound of Formula (I), or a pharmaceutically acceptable salt thereof (*e.g.*. the Tris salt of the compound of Formula (I) and the sodium salt of the compound of Formula (I)) is administered to the subject for 4 consecutive days followed by a 3-day break for the first week of a 28-day or 4-week cycle, where the number of consecutive days of treatment is decreased in at least one of the weeks of a 28-day or 4-week cycle.

In another embodiment, the effective amount of the compound of Formula (I), or a pharmaceutically acceptable salt thereof (*e.g.*. the Tris salt of the compound of Formula (I) and the sodium salt of the compound of Formula (I)) is administered to the subject for 5 consecutive days followed by a 2-day break for the first week of a 28-day or 4-week cycle, where the number of consecutive days of treatment is increased in at least one of the weeks of the 28-day or 4-week cycle.

In another embodiment, the effective amount of the compound of Formula (I), or a pharmaceutically acceptable salt thereof (*e.g.*. the Tris salt of the compound of Formula (I) and the sodium salt of the compound of Formula (I)) is administered to the subject for 5 consecutive days followed by a 2-day break for the first week of a 28-day or 4-week cycle, where the number of consecutive days of treatment is decreased in at least one of the weeks of a 28-day or 4-week cycle.

In one embodiment, the dosing regimen for the lymphoma treatment method described in the foregoing paragraphs comprises administering an effective amount of the compound of Formula (I), or a pharmaceutically acceptable salt thereof (*e.g.,* the Tris salt of the compound of Formula (I) and the sodium salt of the compound of Formula (I)) orally. In one embodiment, the effective amount of the compound of Formula (I) or a pharmaceutically acceptable salt thereof (*e.g.,* the Tris salt of the compound of Formula (I) and the sodium salt of the compound of Formula (I)) is administered as an oral capsule. In another embodiment, the effective amount of the compound of Formula (I), or a pharmaceutically acceptable salt thereof (*e.g.,* the Tris salt of the compound of Formula (I) and the sodium salt of the compound of Formula (I)) is administered as an oral tablet.

In one embodiment, the dosing regimen for the lymphoma treatment method described in the foregoing paragraphs comprises administering an effective amount of the compound of Formula (I), or a pharmaceutically acceptable salt thereof *(e.g.,* the Tris salt of the compound of Formula (I) and the sodium salt of the compound of Formula (I)) once daily (QD). In another embodiment, the dosing regimen for the cancer treatment method described in the foregoing paragraphs comprises administering an effective amount of the compound of Formula (I), or a pharmaceutically acceptable salt thereof (*e.g.,* the Tris salt of the compound of Formula (I) and the sodium salt of the compound of Formula (I)) twice daily (BID).

### Compositions

Also disclosed is a pharmaceutical composition comprising an agent and an anti-cancer therapy, wherein the pharmaceutical composition optionally and additionally comprises an excipient, wherein the anti-cancer agent is represented by Formula (I): 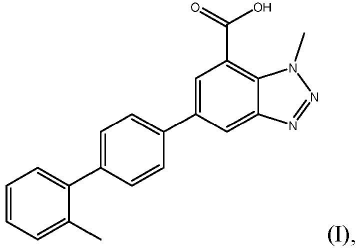 or a pharmaceutically acceptable salt thereof; and wherein the anti-cancer therapy comprises a combination of rituximab, cyclophosphamide, doxorubicin or a pharmaceutically acceptable salt thereof, vincristine or a pharmaceutically acceptable salt thereof, and prednisolone, wherein the lymphoma is diffuse large B-cell lymphoma (DLBCL), mantle cell lymphoma (MCL), or Burkitt lymphoma. In one embodiment, the pharmaceutically acceptable salt thereof of Compound 1 is a tris(hydroxymethyl)aminomethane salt or a sodium salt. In one embodiment, the pharmaceutically acceptable salt thereof of doxorubicin is a hydrochloric acid salt. In one embodiment, the pharmaceutically acceptable salt thereof of vincristine is a sulfate salt.

The anti-cancer agent and/or anti-cancer therapy may be formulated together with a pharmaceutically acceptable carrier, adjuvant, or vehicle into pharmaceutical compositions prior to being administered to a subject.

The term "pharmaceutically acceptable carrier, adjuvant, or vehicle" refers to a carrier, adjuvant, or vehicle that may be administered to a subject, together with the compound of Formula (I), or a pharmaceutically acceptable salt thereof, and/or the anti-cancer therapy, and which does not destroy the pharmacological activity thereof and is nontoxic when administered in doses sufficient to deliver a therapeutic amount of the compound. Those skilled in the art would know how to prepare formulations suitable for administration. The anti-cancer therapy optionally is part of the same formulation for the anti-cancer agent (i.e., the compound of Formula (I), or a pharmaceutically acceptable salt thereof), if it is administered concurrently with the first anti-cancer agent, or, alternatively, can be administered separately.

Pharmaceutically acceptable carriers, adjuvants and vehicles that may be used in the pharmaceutical compositions include, but are not limited to, ion exchangers, alumina, aluminum stearate, lecithin, self-emulsifying drug delivery systems (SEDDS) such as d-α-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as α-, β-, and γ-cyclodextrin, or chemically modified derivatives such as hydroxyalkylcyclodextrins, including 2- and 3-hydroxypropyl-β-cyclodextrins, or other solubilized derivatives may also be advantageously used to enhance delivery of the compound of Formula (I), or a pharmaceutically acceptable salt thereof, and/or a second anti-cancer agent.

The pharmaceutical compositions may be administered orally, parenterally, by inhalation spray, topically, rectally, nasally, buccally, vaginally or via an implanted reservoir, preferably by oral administration or administration by injection. The pharmaceutical compositions may contain any conventional non-toxic pharmaceutically acceptable carriers, adjuvants or vehicles. In some cases, the pH of the formulation may be adjusted with pharmaceutically acceptable acids, bases or buffers to enhance the stability of the formulated compound or its delivery form. The term parenteral as used herein includes subcutaneous, intracutaneous, intravenous, intramuscular, intraarticular, intraarterial, intrasynovial, intrasternal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, or carboxymethyl cellulose or similar dispersing agents which are commonly used in the formulation of pharmaceutically acceptable dosage forms such as emulsions and or suspensions. Other commonly used surfactants such as Tweens or Spans and/or other similar emulsifying agents or bioavailability enhancers which are commonly used in the manufacture of pharmaceutically acceptable solid, liquid, or other dosage forms may also be used for the purposes of formulation.

The pharmaceutical compositions may be orally administered in any orally acceptable dosage form including, but not limited to, capsules, tablets, emulsions and aqueous suspensions, dispersions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions and/or emulsions are administered orally, the active ingredient may be suspended or dissolved in an oily phase is combined with emulsifying and/or suspending agents. If desired, certain sweetening and/or flavoring and/or coloring agents may be added.

The pharmaceutical compositions may also be administered in the form of suppositories for rectal administration. These compositions can be prepared by mixing the compound of Formula (I), or a pharmaceutically acceptable salt thereof, and/or the anti-cancer therapy with a suitable non-irritating excipient which is solid at room temperature but liquid at the rectal temperature and therefore will melt in the rectum to release the active components. Such materials include, but are not limited to, cocoa butter, beeswax and polyethylene glycols.

The amount of active ingredient that may be combined with one or more pharmaceutical excipients to produce a single dosage form will vary depending upon the patient treated and the particular mode of administration. A typical preparation will contain from about 5% to about 95% active compound (w/w). Alternatively, such preparations contain from about 20% to about 80% active compound. In certain embodiments, the pharmaceutical composition comprises between about 10 mg to about 1500 mg of the compound of Formula (I) or a pharmaceutically acceptable salt thereof (based on the weight of the free form of the compound of Formula (I), apart from the weight of any conformer, salt former, water of hydration, solvent of solvation and the like). In some embodiments, the pharmaceutical composition comprises between about 90 mg and about 240 mg; between about 95 mg and about 240 mg; between about 100 mg and about 240 mg; between about 10 mg and about 500 mg; or between about 10 mg and about 1000 mg of the compound of Formula (I) or a pharmaceutically acceptable salt. In some embodiments, the pharmaceutical composition comprises about 10 mg, about 25 mg, about 50 mg, about 75 mg, about 90 mg, about 95 mg, about 100 mg, about 110 mg, about 120 mg, about 125 mg, about 130 mg, about 140 mg, about 150 mg, about 160 mg, about 170 mg, about 175 mg, about 180 mg, about 190 mg, about 200 mg, about 210 mg, about 220 mg, about 225 mg, about 230 mg, about 240 mg, about 250 mg, about 260 mg, about 270 mg, about 275 mg, about 280 mg, about 290 mg, or about 300 mg of the compound of Formula (I) or a pharmaceutically acceptable salt thereof. In some embodiments, the pharmaceutical composition comprises about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, or about 1450 mg, or about 1500 mg. In certain embodiments, the pharmaceutical composition is in the form of an orally acceptable dosage form, such as for example a capsule, and comprises about 50 mg, about 75 mg, about 90 mg, about 95 mg, about 100 mg, about 110 mg, about 120 mg, about 125 mg, about 130 mg, about 140 mg, about 150 mg, about 160 mg, about 170 mg, about 175 mg, about 180 mg, about 190 mg, about 200 mg, about 210 mg, about 220 mg, about 225 mg, about 230 mg, about 240 mg, about 250 mg, about 260 mg, about 270 mg, about 275 mg, about 280 mg, about 290 mg, or about 300 mg of the Tris salt of the compound of Formula (I).

As used herein, the terms "about" and "approximately" when used in combination with a numeric value or range of values mean the value or range of values may deviate to an extent deemed reasonable to one of ordinary skill in the art.

The compound of Formula (I) and formulations thereof can be prepared according to methods described in WO 2014/128669, WO 2019/164794, WO 2020/132471, and US Patent No. 9,630,932.

### EXAMPLES

### Growth inhibition study of Compound 1 with R-CHOP in tumor-bearing mice.

Mice were implanted subcutaneously with 5X10⁶ to 2X10⁷ cells as required for each model. Tumor volumes were monitored twice a week until tumors reach approximately 150 mm³. Once tumor burden was at this level, mice were randomized and treated with the following.

| **Treatment** | **Dose/Regimen** |
|---|---|
| Vehicle | N/A |
| Compound 1 | 100 mg/kg BID (Days 1-23) |
| Rituximab | 20 mg/kg BIW |
| CHOP | C - 30 mg/kg QD (Day 1 and 22) |
| | D - 2.475 mg/kg QD (Day 1 and 22) |
| | V - 0.375 mg/kg QD (Day 1 and 22) |
| | P - 0.15 mg/kg QD (Days 1-5 and Day 22) |
| Compound 1 + R + CHOP | 100 mg/kg + 20 mg/kg + CHOP |

At the study end, tumor tissue, and plasma were collected 1 hr. post final dose of Compound 1. Treatment was well tolerated. **FIGs 1-3** show the percent change in tumor volume for the mouse models treated. As shown in **FIG 1** **-** **FIG 3****,** various models responded significantly with the treatment of Compound 1 with R-CHOP. The magnitude of response varied from complete tumor regression as seen in the Farage (DLBCL) and JVM-2 (MCL) models (see **FIGs 1-2****)** to tumor stasis as seen in the WSUDLCL-2 (DLBCL) model (see **FIG 1****)** for example. A similar result was observed across the multiple subsets of lymphoma tested.

## Claims

1. An anti-cancer agent represented by Formula (1): 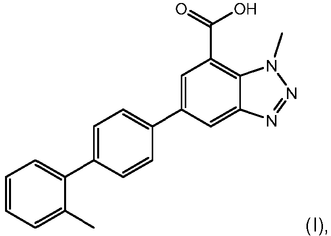 or a pharmaceutically acceptable salt thereof for use in treating lymphoma in combination with an anti-cancer therapy, wherein the anti-cancer therapy comprises a combination of rituximab, cyclophosphamide, doxorubicin or a pharmaceutically acceptable salt thereof, vincristine or a pharmaceutically acceptable salt thereof, and prednisolone, wherein the lymphoma is diffuse large B-cell lymphoma (DLBCL), mantle cell lymphoma (MCL), or Burkitt lymphoma.

2. The anti-cancer agent for use according to claim 1, wherein the lymphoma is mantle cell lymphoma (MCL).

3. The anti-cancer agent for use according to claim 1, wherein the lymphoma is diffuse large B-cell lymphoma (DLBCL).

4. The anti-cancer agent for use according to claim 1, wherein the lymphoma is Burkitt lymphoma.

5. The anti-cancer agent for use according to any one of claims 1 to 4, wherein, in use, the anti-cancer agent and the anti-cancer therapy are administered simultaneously in the same or different formulations.

6. The anti-cancer agent for use according to any one of claims 1 to 5, wherein, in use, the anti-cancer agent and the anti-cancer therapy are administered sequentially.

7. The anti-cancer agent for use according to any one of claims 1 to 6, wherein the anti-cancer agent is the tris(hydroxymethyl)aminomethane salt of the compound of Formula (I).

8. The anti-cancer agent for use according to any one of claims 1 to 7, wherein the anti-cancer agent is the sodium salt of the compound of Formula (1).

9. The anti-cancer agent for use according to any one of claims 1 to 8, wherein the doxorubicin is doxorubicin hydrochloride.

10. The anti-cancer agent for use according to any one of claims 1 to 9, wherein the vincristine is vincristine sulfate.

## Patentansprüche

1. Antikrebsmittel, dargestellt durch Formel (I): 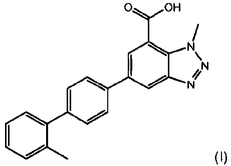 oder ein pharmazeutisch unbedenkliches Salz davon zur Verwendung bei der Behandlung von Lymphom in Kombination mit einer Krebstherapie, wobei die Krebstherapie eine Kombination aus Rituximab, Cyclophosphamid, Doxorubicin oder einem pharmazeutisch unbedenklichen Salz davon, Vincristin oder einem pharmazeutisch unbedenklichen Salz davon und Prednisolon umfasst, wobei es sich bei dem Lymphom um ein diffuses großzelliges B-Zell-Lymphom (DLBCL), ein Mantelzelllymphom (MCL) oder ein Burkitt-Lymphom handelt.

2. Antikrebsmittel zur Verwendung nach Anspruch 1, wobei es sich bei dem Lymphom um ein Mantelzelllymphom (MCL) handelt.

3. Antikrebsmittel zur Verwendung nach Anspruch 1, wobei es sich bei dem Lymphom um ein diffuses großzelliges B-Zell-Lymphom (DLBCL) handelt.

4. Antikrebsmittel zur Verwendung nach Anspruch 1, wobei es sich bei dem Lymphom um ein Burkitt-Lymphom handelt.

5. Antikrebsmittel zur Verwendung nach einem der Ansprüche 1 bis 4, wobei das Antikrebsmittel und die Krebstherapie bei der Verwendung gleichzeitig in der gleichen oder verschiedenen Formulierungen verabreicht werden.

6. Antikrebsmittel zur Verwendung nach einem der Ansprüche 1 bis 5, wobei das Antikrebsmittel und die Krebstherapie bei der Verwendung nacheinander verabreicht werden.

7. Antikrebsmittel zur Verwendung nach einem der Ansprüche 1 bis 6, wobei es sich bei dem Antikrebsmittel um das Tris(hydroxymethyl)aminomethansalz der Verbindung von Formel (I) handelt.

8. Antikrebsmittel zur Verwendung nach einem der Ansprüche 1 bis 7, wobei es sich bei dem Antikrebsmittel um das Natriumsalz der Verbindung von Formel (I) handelt.

9. Antikrebsmittel zur Verwendung nach einem der Ansprüche 1 bis 8, wobei es sich bei dem Doxorubicin um Doxorubicin-Hydrochlorid handelt.

10. Antikrebsmittel zur Verwendung nach einem der Ansprüche 1 bis 9, wobei es sich bei dem Vincristin um Vincristinsulfat handelt.

## Revendications

1. Agent anticancéreux représenté par la formule (I) : 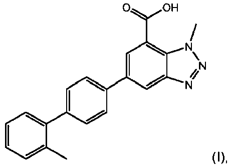 ou un sel pharmaceutiquement acceptable de celui-ci destiné à être utilisé dans le traitement du lymphome en association avec une thérapie anticancéreuse, dans lequel la thérapie anticancéreuse comprend une combinaison de rituximab, de cyclophosphamide, de doxorubicine ou d'un sel pharmaceutiquement acceptable de celui-ci, de vincristine ou d'un sel pharmaceutiquement acceptable de celui-ci, et de prednisolone, dans lequel le lymphome est un lymphome diffus à grandes cellules B (LDGCB), un lymphome à cellules du manteau (LCM) ou un lymphome de Burkitt.

2. Agent anticancéreux destiné à être utilisé selon la revendication 1, dans lequel le lymphome est un lymphome à cellules du manteau (LCM).

3. Agent anticancéreux destiné à être utilisé selon la revendication 1, dans lequel le lymphome est un lymphome diffus à grandes cellules B (LDGCB).

4. Agent anticancéreux destiné à être utilisé selon la revendication 1, dans lequel le lymphome est un lymphome de Burkitt.

5. Agent anticancéreux destiné à être utilisé selon l'une quelconque des revendications 1 à 4, dans lequel, lors de son utilisation, l'agent anticancéreux et la thérapie anticancéreuse sont administrés simultanément dans les mêmes formulations ou dans des formulations différentes.

6. Agent anticancéreux destiné à être utilisé selon l'une quelconque des revendications 1 à 5, dans lequel, lors de son utilisation, l'agent anticancéreux et la thérapie anticancéreuse sont administrés de manière séquentielle.

7. Agent anticancéreux destiné à être utilisé selon l'une quelconque des revendications 1 à 6, dans lequel l'agent anticancéreux est le sel de tris(hydroxyméthyl)aminométhane du composé de formule (I).

8. Agent anticancéreux destiné à être utilisé selon l'une quelconque des revendications 1 à 7, dans lequel l'agent anticancéreux est le sel de sodium du composé de formule (I).

9. Agent anticancéreux destiné à être utilisé selon l'une quelconque des revendications 1 à 8, dans lequel la doxorubicine est du chlorhydrate de doxorubicine.

10. Agent anticancéreux destiné à être utilisé selon l'une quelconque des revendications 1 à 9, dans lequel la vincristine est du sulfate de vincristine.
